# EUROPEAN PATENT APPLICATION

(11) **EP 4 523 610 A1**
(43) Date of publication of application: **19.03.2025**
(21) Application number: 23382930.8
(22) Date of filing: 13.09.2023
(51) Int. Cl.: A61B 5/00

(54) **CATHETER-BASED BIREFRINGENCE MAPPING FOR ABLATION PROCEDURE**

(71) Applicant: Medlumics S.L., 28760 Tres Cantos, Madrid (ES)
(72) Inventor: DUPERRON, Matthieu, 28760 Madrid (ES); HERRANZ ARAGONCILLO, David, 28760 Madrid (ES); BAILLEUL, Christophe, 28760 Madrid (ES)
(74) Representative: DTS Patent- und Rechtsanwälte PartmbB

(57) **Abstract**

Described herein are systems, methods, and computer-readable media for catheter-based birefringence mapping for an ablation procedure. A system includes a catheter that includes optical fibers coupled to a computing device that includes a processer that may create a three-dimensional map that displays optical properties of a tissue using optical measurements, specifically birefringence measurements, and a mapping system.

## Description

### BACKGROUND

### Field

Aspects of the present disclosure relate to components, systems, and methods for using catheter and console devices for collecting birefringence measurements of tissue and mapping the birefringence measurements on a three-dimensional map of the tissue.

### Background

Ablation is a medical technique for producing tissue necrosis. It is used to help treat different pathologies including cancer, Barret's esophagus, or cardiac arrhythmias, among others. The application of ablation energy produces denaturation of the large biomolecules, including proteins such as collagen, myosin, or elastin. Traditionally, ablation is performed by applying an alternating potential to the tip of a catheter that is placed in contact with the tissue to be treated within the patient's body.

The ablation effect depends on several factors, including applied electrical power, quality of the electrical contact, local tissue properties, presence of blood flow close to the tissue surface, and the effect of irrigation. Because of the variability of these parameters, it may be difficult to obtain consistent results and understand ablation effects in tissue using current systems and methods for ablation.

Accordingly, such systems and methods may be limited because of the difficulties and challenges in assessing the results of ablation in tissue, such as identifying a lesion formed in the tissue and determining various properties of the lesion through the catheter.

### BRIEF DESCRIPTION OF THE DRAWINGS

The accompanying drawings, which are incorporated herein and form a part of the specification, illustrate the present invention and, together with the description, further explain the principles of the invention and to enable a person skilled in the pertinent art to make and use the invention.
FIG. 1 illustrates an example diagram of a system for ablation and lesion prediction, according to aspects of the present disclosure.
FIG. 2A illustrates an example diagram of a catheter, according to aspects of the present disclosure.
FIG. 2B illustrates a diagram of an example distal section of a unipolar catheter, according to aspects of the present disclosure.
FIG. 2C illustrates a diagram of an example distal section of a bipolar catheter, according to aspects of the present disclosure.
FIGs. 3A and 3B illustrate cross sections of a catheter, according to embodiments of the present disclosure.
FIG. 4A illustrates a diagram of an example OCT and/or OCR system with multiple optical components, according to embodiments of the present disclosure.
FIG. 4B illustrates an example block diagram showing a full chain of integration of data acquisition of the optical signals, according to aspects of the present disclosure.
FIG. 5 illustrates an example method for mapping optical measurements on a three-dimensional map, according to aspects of the present disclosure.
FIG. 6 illustrates an example method for mapping birefringence measurements on a three-dimensional map, according to aspects of the present disclosure.
FIG. 7 illustrates an example of a three-dimensional map that displays birefringence measurements, according to aspects of the present disclosure.
FIG. 8 illustrates a block diagram of example components of a computer system, according to aspects of the present disclosure.
FIG. 9 illustrates an example of the placement of orthogonal skin patches, according to aspects of the present disclosure.

Aspects of the present invention will be described with reference to the accompanying drawings. The drawing in which an element first appears is typically indicated by the leftmost digit(s) in the corresponding reference number.

### DETAILED DESCRIPTION

During ablation (e.g., radiofrequency ablation (RFA), pulsed field ablation (PFA), laser ablation, ultrasonic ablation, microwave ablation, etc.) procedures, electrophysiologists (EP) use a catheter to ablate specific locations of an organ or vessel, such as a heart, to create transmural lines of ablation that electrically isolate different parts, sections, or areas of the organ. To guide the ablation, an EP may use an anatomical and voltage mapping system to locate the tip of the catheter in a cavity of the organ and perform the ablation. At the start of a procedure to perform an ablation, the EP uses a catheter to sweep the cavities of the organ using the tip of the catheter. The tip of the catheter is used as a reference for the structural mapping of the organ and an electrical sensor to create an electrophysiological map of the organ. The structural and voltage mapping are used by the EP to determine where the ablation points should be performed and to evaluate the success of the ablations.

However, the use of electrophysiological signals gives an incomplete representation of the success of an ablation because it focuses on electrophysiological signals propagating at the surface of the organ tissue and does not assess the effect of the ablation on the depth of the tissue. For example, RFA is often used in cardiac ablations, but its incompleteness leads to a high level of Atrial Fibrillation recurrence with recurrence rates ranging from 25% to more than 60% depending on patient characteristics. In PFA, electrophysiological signals cannot be used to assess the success of an ablation since even sub-therapeutic ablations cause the abolition of electrical activity for prolonged periods of time, meaning that no direct measurement of ablation success can be provided to the EP. For these reasons, a system for mapping the effect of ablation on the depth of the tissue using optical measurements may improve the results of ablation on an organ.

An improved system for mapping the effect of ablation on the depth of a tissue can be accomplished through mapping the birefringence of the tissue. Birefringence is a physical property of anisotropic media (e.g., organized tissues). Areas of low birefringence are associated with unorganized tissue (isotropic medium), where the probability of such tissue propagating the electrical signal is low. An EP may be able to use the location of low birefringence areas to guide an ablation strategy during an ablation procedure because isotropic tissues are related to physical specificities such as ablated tissue, fibrosis and/or scars, and tissues with venous capillaries. Additionally, surrounding structures that modify the shape of an ablation lesion due to the non-uniform propagation of the electromagnetic field may also be characterized by the EP by mapping birefringence. A birefringence map can be used to guide where the location of ablation is performed during the procedure. A birefringence map may also track the decrease of anisotropy of the tissue induced by the ablation over time during the procedure, such as over the entire procedure time. The birefringence map may be used instead of or as a complement to electrophysiological information.

Although specific configurations and arrangements are discussed, it should be understood that this is done for illustrative purposes only. A person skilled in the pertinent art will recognize that other configurations and arrangements can be used without departing from the spirit and scope of the present disclosure. It will be apparent to a person skilled in the pertinent art that this disclosure can also be employed in a variety of other applications.

It is noted that references in the specification to "one aspect," "an aspect," "an example aspect," etc., indicate that the aspect described may include a particular feature, structure, or characteristic, but every aspect may not necessarily include the particular feature, structure, or characteristic. Moreover, such phrases do not necessarily refer to the same aspect. Further, when a particular feature, structure, or characteristic is described in connection with an aspect, it would be within the knowledge of one skilled in the art to use such feature, structure, or characteristic in connection with other aspects whether or not explicitly described.

It should be noted that although this application may refer specifically to cardiac ablation, the aspects described herein may target other pathologies as well, along with additional energy sources for ablation, including but not limited to cryogenic, radiofrequency (RF), microwave, laser, ultrasound, and pulsed electric fields. The principles of using energy to treat other pathologies are similar, and therefore the techniques used to apply the energy are similar.

Herein, the terms "electromagnetic radiation," "light," and "beam of radiation" are all used to describe the same electromagnetic signals propagating through the various described elements and systems.

An example catheter and optical analysis system useful in aspects of the present invention is described in U.S. Patent No. 11,523,740, which is incorporated by reference herein in its entirety.

### Example Mapping System and Console Aspects

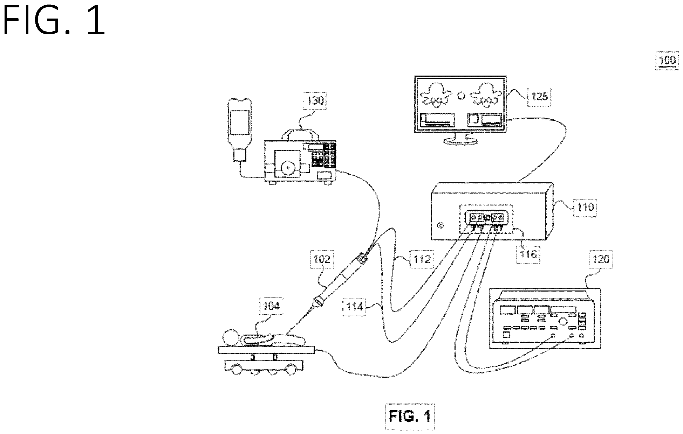

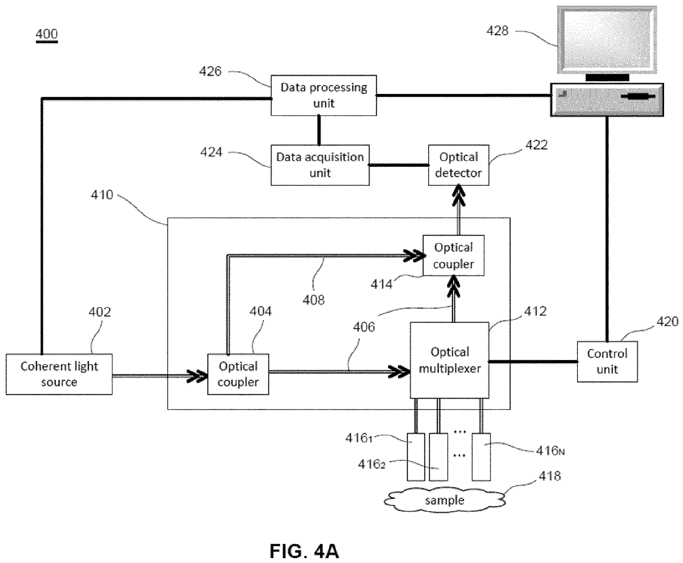

In some aspects, an electroanatomical mapping system may be used to anatomically reconstruct an organ of an animal into a virtual three-dimensional (3D) map. In some aspects, the mapping system may be an independent catheter, console, and/or system that collects position information to generate the 3D map. In some aspects, the mapping system may be integrated into a catheter system that collects optical measurements (e.g., catheter system 100 in FIG. 1 and optical system 400 in FIG. 4A). Such a mapping system may be, for example, a commercial system such as an EnSite^{™} Precision, EnSite^{™} Automap Module, Biosense CARTO^{™} system, etc. The mapping system may be an invasive or non-invasive system.

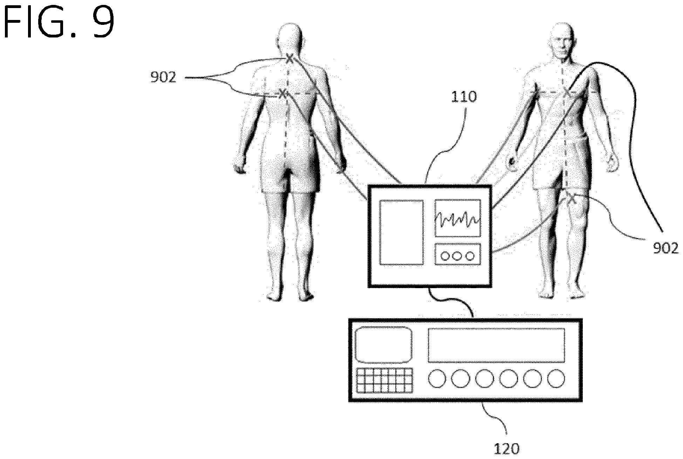

In order to generate the 3D map, an electrical and/or magnetic field may be created around a patient. For example, the mapping system may use orthogonal skin patches (e.g., three electrode pairs form three orthogonal axes (x-y-z) with the organ of interest in the center) on a patient that create an electrical field centered on the patient's thorax. FIG. 9 illustrates an example of the placement of such orthogonal skin patches 902. In some aspects, the skin patches may be placed on the patient's back, chest, and leg. In some aspects there may be 3, 4, 5, 6, 7, or more skin patches positioned on a patient.

In another example, the mapping system uses at least three coils positioned around a patient to generate a magnetic field. In some aspects, both electrical and magnetic fields may be generated around the patient.

A catheter with capability to collect electrophysiological (EP) information may be inserted into the organ of interest. The catheter may have a sensor at its tip that detects the electrical and/or magnetic fields created by the external field generators. The location of the catheter may be determined based on the strength of the field(s) detected by the catheter sensor. An orientation of the catheter tip may also be determined if there are multiple sensors in the catheter, or if the catheter sensor is a directional sensor. In some aspects, the catheter tip does not contain a sensor but instead contains an emitter, whose signal is detected by external sensors such as electromagnetic sensors, acoustic sensors, and the like. The location of the catheter tip can then be determined by triangulating the signals received by the external sensors.

As the catheter is positioned at or swept across various points on the surface of the organ (such as the heart), the catheter tip's location can be tracked, and the collected locations used to generate a virtual 3D model of the organ. In some aspects, the mapping system collects location data (e.g., coordinates) for hundreds or thousands of points within and/or across the surface of an organ. In general, the greater the number of locations collected, the more accurate the virtual 3D model will be. If the catheter also takes EP measurements while it is being tracked, those EP measurements may be overlaid onto the 3D model to create an EP map. For example, according to aspects described herein, the 3D model may be used to display specific measurements of the organ on a 3D model of the organ, including one or more of impedance/voltage, temperature, birefringence, etc.

The 3D model may be generated in real-time or near real-time such that an electrophysiologist may perform analysis during a procedure. Alternatively, the 3D model may be generated in advance of a procedure such that measurements taken during the procedure are mapped against the pre-existing 3D model. The 3D model may be displayed on any computing system that includes a display. In some aspects, a single catheter tip contains one or more sensors that give the single catheter both mapping and analysis capabilities. In some aspects, the catheter is an ablation catheter that also has ablation capabilities. In some aspects, the mapping catheter is separate from the ablation and/or analysis catheter, with the ablation and/or analysis catheter containing a sensor or emitter that can nonetheless be used to locate the catheter tip within the same coordinate system as used with the mapping catheter.

### Example Catheter System and Console Aspects

FIG. 1 illustrates an example diagram of a system 100 for performing ablation and generating a birefringence map, according to aspects of the present disclosure. The system 100 includes catheter 102, console 110, signal generator 120, display 125, and irrigation pump 130. The catheter 102, console 110, signal generator 120, display 125, and irrigation pump 130 may be communicatively coupled together via wired and/or wireless connections. In some aspects, console 110 may receive electrical and/or magnetic field information from a plurality of field generators or sensors such as the patches 902 illustrated in FIG. 9, described above. In some aspects, catheter 102 may represent an exemplary aspect of catheter 200 as described below. In some aspects, a distal section of catheter 102 is positioned at a portion of tissue in patient 104. It is understood that the aspects described herein may be used in vivo and/or in vitro.

In some aspects, catheter 102 may be positioned at a portion of tissue subject to ablation using energy generated by signal generator 120. In some aspects, signal generator 120 may be an electronic device configured to generate radiofrequency (RF), cryogenic, or electroporation (e.g., pulsed electric field (PFA)) signals for ablation. The signal generator 120 may be coupled to catheter 102 directly or via the console 110 and may send energy to catheter 102 to ablate a portion of tissue at a selected tissue site. In some aspects, the portion of tissue may include myocardial tissue, cardiac muscle tissue, skeletal tissue, or the like. Energy may be applied to the portion of tissue through optical view ports in the distal section of catheter 102. After applying the energy, structural changes in the tissue may be observed by acquiring optical signals via one or more optical viewports of catheter 102.

Console 110 may comprise a computing device configured to acquire the optical signals from catheter 102 and analyze the optical signals to detect changes in optical properties of the tissue. In some aspects, console 110 may include hardware (e.g., circuits), firmware, software, or any combination thereof to perform analysis of the optical signals and generate a birefringence map as described herein. In some aspects, console 110 may send light through an optical circuit within itself and the catheter 102 and into the tissue to monitor scar progression, contact between the tissue and catheter 102, and other characteristics of the tissue. In some aspects, console 110 may be referred to herein as a control console, a processing device, and/or a controller. Console 110 may be coupled to display 125, which may present results from the optical signal analysis and lesion predictions and allow a user to select/view, modify, and/or control parameters related to the operation of catheter 102, console 110, signal generator 120, and/or irrigation pump 130.

In some aspects, irrigation pump 130 may be coupled to catheter 102 via a tubing. In some aspects, irrigation pump 130 may allow for fluid to be pumped through the tubing and released at the tissue site through catheter 102 (e.g., through optical view ports or through separate irrigation slits at the distal section of catheter 102). Fluid from the irrigation pump 130 may cool the distal section of catheter 102 and the surrounding tissue during ablation, and also flush away any debris during and/or after ablation.

In some aspects, catheter 102 may be coupled to console 110 via one or more optical connections 112 and one or more electrical connections 114. Optical connections 112 may include single mode optical fibers and/or multimode optical fibers that allow acquisition and/or transmission of optical signals to and from catheter 102 and console 110 for further analysis. Electrical connections 114 may include wiring, pins, and/or components used for supplying power and energy from signal generator 120 to catheter 102 for ablation.

In some aspects, the optical and electrical connections 112, 114 may be connected to console 110 via a communication interface 116. Communication interface 116 may allow for transmission of various signals (e.g., optical and electrical signals) between catheter 102 and console 110. In some aspects, the communication interface 116 may include a connector that facilitates proper alignment of optical fibers between the catheter 102 and console 110. In some aspects, the connector design may include both electrical and optical extension lines.

### Exemplary Catheter Aspects

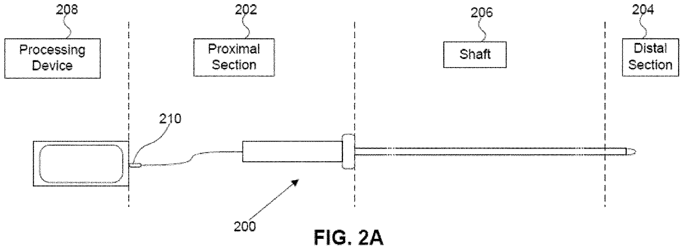

FIG. 2A illustrates a catheter 200 according to aspects of the present disclosure. Catheter 200 includes a proximal section 202, a distal section 204, and a shaft 206 coupled between proximal section 202 and distal section 204. In an aspect, shaft 206 includes one or more radiopaque markers for navigation purposes. In one aspect, catheter 200 includes a communication interface 210 between catheter 200 and a processing device 208. Communication interface 210 may include one or more optical fibers and connectors between processing device 208 and catheter 200. In other examples, communication interface 210 may include an interface component that allows wireless communication, such as Bluetooth, Wi-Fi, cellular, and the like, to communicate with the catheter 200 or other processing components in a catheter system.

Proximal section 202 may house various electrical and optical components used in the operation of catheter 200. In some embodiments, an optical source may be included within proximal section 202 to generate a source beam of radiation for optical evaluation. In some embodiment, the optical source can be an external optical source coupled to the proximal section via optical connections 112 in FIG. 1. The optical source may include one or more lasers, laser diodes, light emitting diodes (LEDs), or other kinds of optical coherent source. The beam of radiation generated by the optical source may have a wavelength within the infrared range. In one example, the beam of radiation has a central wavelength between about 1000 nm and 1600 nm. The optical source may be designed to output a beam of radiation at only a single wavelength, or it may be a swept source and be designed to output a range of different wavelengths. The generated beam of radiation may be guided towards distal section 204 via the optical transmission medium connected between proximal section 202 and distal section 204 within shaft 206. Some examples of optical transmission media include single-mode optical fibers and/or multimode optical fibers. In one embodiment, the electrical transmission medium and the optical transmission medium are provided by the same hybrid medium allowing for both electrical and optical signal propagation.

In some aspects, proximal section 202 may include a second optical source, such as a laser energy source, to generate laser energy that is applied at distal section 204 for tissue ablation. In some aspects, proximal section 202 may include a non-optical energy source for generating non-optical energy, such as RF energy, to be applied at distal section 204 for tissue ablation. In some aspects, proximal section 202 may include both a second optical and a non-optical energy source for the selection of energy to be applied at distal section 204. In some aspects, processing device 208 may include one or more components, such as detectors, electronics, and/or other components of an optical circuit/system as described herein. In other aspects, one or more components, such as detectors, electronics, and/or other components of an optical circuit/system may be included in the proximal section 202.

In an embodiment, proximal section 202 includes one or more components of an interferometer in order to perform coherence interferometry using the light generated from the optical sources. Due to the nature of interferometric data analysis, in an embodiment, the optical transmission medium used for guiding the light to and from distal section 204 does not affect the state and degree of light polarization. In another embodiment, the optical transmission medium can affect the polarization in a constant and reversible way. In some embodiments, catheter 200 may include an optical circuit with one or more elements configured to conduct optical spectroscopy. In such embodiments, at least part of the optical path may be made up of multi-mode optical transmission media (e.g., multi-mode optical fiber).

Proximal section 202 may include further interface elements with which a user of catheter 200 can control the operation of catheter 200. For example, proximal section 202 may include a deflection control mechanism that controls a deflection angle of distal section 204. The deflection control mechanism may include a mechanical movement of an element on proximal section 202, or the deflection control mechanism may use electrical and/or mechanical connections to control the movement of distal section 204. Proximal section 202 may include various buttons or switches that allow a user to control when the beams of radiation are transmitted from distal section 204, allowing for the acquisition of optical data. In some embodiments, proximal section 202 may include a deflection control mechanism for controlling one or more pull wires that are coupled to the distal section 204. In some embodiments, thee deflection control mechanism and one or more pull wires allow for steering of the distal section of catheter 200 in order to maneuver within and target specific tissue regions for ablation.

Distal section 204 includes a plurality of optical viewports. In some embodiments, the plurality of optical viewports may be referred to herein as orifices in the catheter tip. In an embodiment, one or more of the optical viewports are machined into the outer body of distal section 204. The optical view ports are distributed over the outside of distal section 104, resulting in a plurality of distinct viewing directions. In some embodiments, the optical view ports may transmit and collect light (e.g., optical signals) at various angles from the distal section 204. In an embodiment, each of the plurality of viewing directions are substantially non-coplanar. The optical viewports may also be designed with irrigation functionality to cool distal section 204 and surrounding tissue during ablation.

In an aspect, distal section 204 may include one or more position sensors that represents the position and/or orientation of distal section 204. For example, distal section 204 may include an electromagnetic position sensor to generate an electromagnetic signal that, when received by an external receiver, can be mapped to a coordinate system to locate the position of the catheter tip within the body. As a further example, a 3-D mapping system, such as an electroanatomic monitoring (EAM) system, based on impedance, and/or a magnetic imaging resonance (MRI) system, may be used to generate an electrophysiological map of an organ, such that the position of the catheter may be identified with reference to the electrophysiological map. Example methods of generating an electrophysiological map may be found in U.S. Patent Nos. 6,226,542 and 6,892,091, the disclosures of which are incorporated by reference herein.

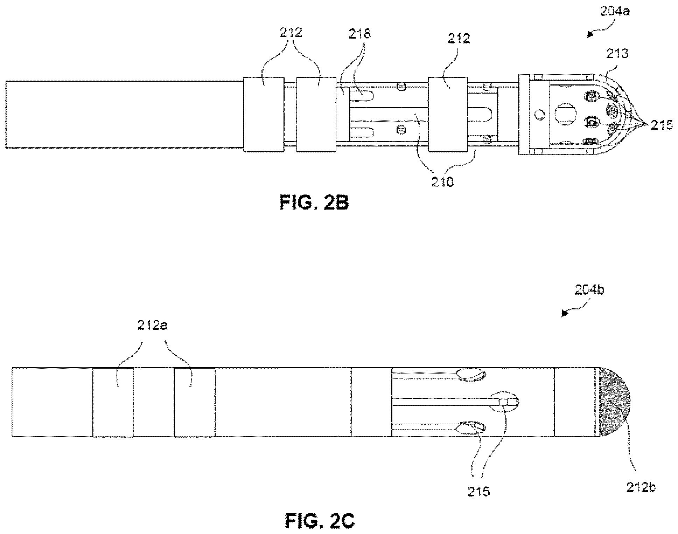

In some embodiments, a unipolar catheter can include a distal section having electrodes with one polarity. FIG. 2B illustrates a diagram of an example distal section of a unipolar catheter 204a, according to embodiments of the present disclosure. In some embodiments, the distal section of catheter 204a in FIG. 2B may represent an example embodiment of distal section 204 of catheter 200 shown in FIG. 2A. The distal section of catheter 204a includes a plurality of electrodes 212, ablation cap 213, a plurality of optical ports 215, one or more pull wire components 218, and irrigation tubing 210. In some embodiments, the distal section of the catheter 204 may have a single electrode or multiple electrodes 212 having a same polarization to deliver ablation energy. In some embodiments, an electrode 212 may be located at the very tip of distal section 204. In some embodiments, PFA energy may be used, and the electrode 212 configuration may be configured to deliver monophasic or biphasic pulse applications. In some embodiments, ablation cap 213 may also be an electrode and may be metallic. In some embodiments, ablation cap 213 may be referred to as a distal cap. In some embodiments, the plurality of optical ports 215 may be referred to herein as a plurality of optical view ports. In some embodiments, the pull wire components 218 may include an anchor and/or other components for allowing steering of the distal section of catheter 204 in order to maneuver within and target specific tissue regions for ablation. In some embodiments, irrigation tubing 210 may allow fluid to be guided along the catheter tip to cool tissue.

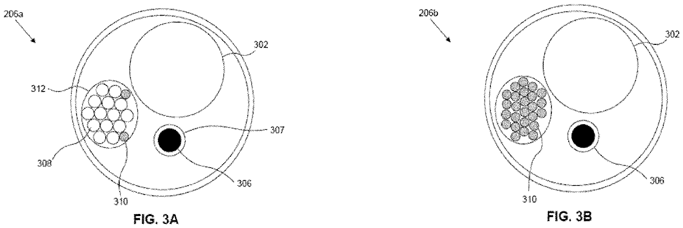

In some embodiments, the ablation cap 213 may include multiple optical ports 215, which may serve as orifices for optical ablation and also as optical windows or view ports for light beams from a plurality of optical fibers in the catheter. In some embodiments, optical ports 215 can be distributed in a non-planar manner on a dome-shaped surface of ablation cap 213. In some embodiments, optical fibers (for example, optical transmission media 310 in FIGs. 3A and 3B) may be directed through the catheter shaft to lenses on the distal section of the catheter. In some embodiments, the optical fibers may be connected to the lenses by wafer-based wave-guide circuits that define the optical components at the catheter tip. In other embodiments, the optical fibers in the catheter tip may connect directly to the lenses, which focus the light into the tissue through the plurality of optical ports 215. In some embodiments, the lenses may be silicon or formed from another optically transparent material. In some embodiments, the lenses may also be coated to reduce reflections at interfaces or to allow optical index differences with surrounding tissue, blood, or fluid media.

In some embodiments, a bipolar catheter can include a distal section having electrodes with two polarities. FIG. 2C illustrates a diagram of an example distal section of a bipolar catheter 204b, according to embodiments of the present disclosure. In some embodiments, the distal section of catheter 204b in FIG. 2C may represent an example embodiment of distal section 204 of catheter 200 shown in FIG. 2A. The distal section of catheter 204b includes a plurality of optical ports 215, one or more of a plurality of electrodes 212a and a tip 212b. In some embodiments, electrodes 212a can have a polarization opposite to each other. In some embodiments, electrodes 212a may be located at a middle region of distal section 204b. In some embodiments, tip 212b may be located at the very tip of distal section 204b. In some embodiments, PFA energy may be used, and electrodes 212a may be configured to deliver monophasic or biphasic pulse applications.

In some embodiments, the catheter tip may include passive and fixed number of optics components (e.g., 15 fibers with 15 lenses attached), without any mechanical switching or scanning devices in the catheter itself. In some embodiments, movement or rotation of optical elements may allow for scanning in different directions in the tissue. In some embodiments, the plurality of optical ports or viewports in the catheter may have various orientations in the catheter tip, in which each output beam directed from each viewport in the catheter may face a different direction. For example, one output beam may be directed forward, seven output beams may be directed at 45° with respect to tissue, and seven output beams may be directed at 90° with respect to tissue. In some embodiments, there may be any number of beams, viewports, orientations of the viewports in the catheter tip.

FIGs. 3A and 3B illustrate cross-section views of shaft 206, according to embodiments of the present disclosure. Shaft 206 may include all of the elements interconnecting proximal section 202 with distal section 204. Shaft 206a illustrates an embodiment that houses multiple channels/lumens, including an irrigation channel 302, a cabling channel 312, and a channel for deflection mechanisms 307. Through these channels 307, 312, 302, deflection mechanism 306, electrical connections 308, and optical transmission medium 310, and cooling fluid may be at least partially housed or transported. In some configurations, a protective cover wrapped around both electrical connections 308 and optical transmission media 310 may be used. In other embodiments, optical transmission media 310 and components may be located within a protective cover that is separate from the protective cover in which the electrical connections 308 is housed. In some embodiments, electrical connections 308 may be used to provide signals to optical modulating components located in distal section 204. In some embodiments, electrical connections 308 may connect to electrodes 212 of FIG. 2B to deliver ablation energy. In some embodiments, one or more optical transmission media 310 guide light generated from the optical source (exposure light) towards distal section 204, while another subset of optical transmission media 310 guides light returning from distal section 204 (scattered or reflected light) back to proximal section 202. In another example, the same one or more optical transmission media 310 guides light in both directions. In some embodiments, the optical transmission medium 310 comprises one or more single-mode optical fibers and/or multimode optical fibers.

Irrigation channel 302 may be a hollow tube used to guide cooling fluid towards distal section 204. Irrigation channel 302 may include heating and/or cooling elements disposed along the channel to affect the temperature of the fluid. In another embodiment, irrigation channel 302 may also be used as an avenue for drawing fluid surrounding distal section 204 back towards proximal section 302.

Deflection mechanism 306 may include electrical and/or mechanical elements designed to provide a signal to distal section 204 in order to change a deflection angle of distal section 204. The deflection system enables guidance of distal section 204 by actuating a mechanical control placed in proximal section 202, according to an embodiment. This system may be based on a series of aligned and uniformly spaced cutouts in shaft 206 aimed at providing unidirectional deflection of distal section 204, in combination with a wire which connects the deflection mechanism control in proximal section 202 with the catheter tip at distal section 204. In this way, a certain movement of the proximal section may be projected to the distal section. Other embodiments involving the combination of several control wires attached to the catheter tip may enable the deflection of the catheter tip along different directions.

FIG. 3B illustrates a cross-section of shaft 206b. Shaft 206b depicts an embodiment having most of the same elements as shaft 206a from FIG. 3A, except that there are no electrical connections 308. Shaft 206b may be used in situations where modulation (e.g., multiplexing) of the generated beam of radiation is performed in proximal section 202 or in distal section 204.

Referring back to FIGs. 2B and 2C, when performing an ablation treatment, ablation energy (e. g., PFA energy) may be delivered to electrodes 212 or 212a in distal section 204 of the catheter 200 and be applied to a target tissue to be treated. A pulsed electric field vector (PEF vector) of the PFA energy may be affected by various factors, such as geometrical shapes of the electrodes and relative positions of the electrodes. In some embodiments, the effect of the ablation energy applied on the target tissue and surroundings depends on various factors, and parameters determining the amount of ablation energy need to be carefully chosen, such as the amplitude and the duration of the pulsed electric field, in order to effectively treat the target tissue while minimizing the damage to surrounding tissues. In particular, the fiber alignment of the target tissue with respect to the PEF is an important factor to be considered for determining the parameters of the PFA energy when performing an ablation treatment.

Specifically, to reach the electroporation irreversibility barrier, the intensity of the applied electric field must be above a certain threshold. As the applied electric field is a vector, its orientation with respect to the cell membrane is important in cardiac cells having an elongated shape. It is therefore useful to know the orientation of the muscle fibers with respect to the applied field to achieve the optimized electroporation thresholds. Having the capability to directly assess the orientation of the muscle fibers with respect to the applied field would enable clinicians to conduct safer, more reliable and durable ablation treatment.

The information about the orientation of the muscle fibers with respect to the applied field is not only useful at the endocardial surface with which the electrodes are placed in contact, but also through the depth of the tissue. The implementation of ablation requires consistent and accurate detection of the tissue to be treated and the surrounding environment. In some cases, optical coherence interferometry, such as OCT or OCR, can be used for measuring various parameters of the tissue samples, such as the tomography and spectra of absorption, reflection, and birefringence. In some cases, these parameters are related to the orientation of the muscle fibers of the tissue, and vary during the process of the ablation treatment, requiring real time detection and analysis to provide reliable information and guidance to the ablation treatment.

### Example Optical System and Console Aspects

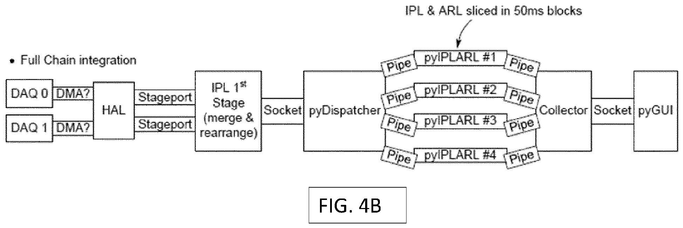

FIG. 4 illustrates a diagram of an example system 400 to perform OCT and/or OCR with multiple optical components, according to embodiments of the present disclosure. A coherent light source 402 provides a beam of coherent light. In some embodiments, coherent light source 402 can be an akinetic swept source or a VCSEL with tunable wavelength (for example, via a MEMS tunable filter). In some embodiments, coherent light source 402 can provide a beam of coherent light with a coherence length (single path in air). For example, the beam of coherent light provided by coherent light source 402 can have a coherence length of 0.1 mm to 1.0 mm, 1.0 mm to 1.0 cm, 1.0 cm to 10 cm, or greater than 10 cm. In some embodiments, coherent light source 402 can be a sweep source that generates a beam of coherent light having a wavelength periodically swept within a wavelength range around a central wavelength at a wavelength sweeping frequency. In some embodiments, the central wavelength can be infrared. In some embodiments, the central wavelength can be between 1000 nm and 1600 nm. For example, the central wavelength of the beam of coherent light can be about 1310 nm. In some embodiments, the wavelength range swept around the central wavelength can be between 30 nm and 70 nm. For example, the wavelength range swept around the central wavelength can be about 55 nm. In some embodiments, the wavelength sweeping frequency can be between 20 kHz and 100 kHz. For example, the wavelength sweeping frequency can be about 50 kHz. In some embodiments, coherent light source 402 can be an internal light source included in console 110 (as shown in FIG. 1) and coupled to optical transmission medium 310 (as shown in FIGs. 3A and 3B) of the catheter.

The beam of coherent light generated by coherent light source 402 is coupled to an interferometer 410. Interferometer 410 includes a first optical coupler 404 that couples a first portion of the beam of coherent light to a reference arm 408 and a second portion of the beam of coherent light to a sample arm 406. In some embodiments, sample arm 406 can be coupled to an optical multiplexer 412, which guides the second portion of the beam of coherent light into one or more optical components 416₁ - 416_{N}. Each of optical components 416₁ - 416_{N} includes an optical path that guides the second portion of the beam of coherent light to a location on a sample tissue 418 to be inspected. Sample 418 can be a healthy tissue, fibrotic tissue, a scar, tissue under an ablation process, or a test sample. Each of optical components 416₁ - 416_{N} can deliver the second portion of the beam of coherent light through one of the optical ports at the distal section of the catheter (for example, optical ports 215 in FIG. 2B). After illumination by the coherent light, a plurality of optical signals can return from sample 418, each corresponding to optical components 416₁ - 416_{N}. The plurality of optical signals depends on the condition of sample 418 and are correlated to the beam of coherent light. The plurality of optical signals are collected back by optical components 416₁ - 416_{N} and returned to sample arm 406 via optical multiplexer 412, and then combined with the first portion of the beam of coherent light in reference arm 408 by a second optical coupler 414. In some embodiments, a control unit 420 can be used to control optical multiplexer 412 to select one or more optical components 416₁ - 416_{N}, to which the second portion of the beam of coherent light is coupled. Control unit 420 can be connected to a computer 428 to receive commands from and transmit data to computer 428.

In some embodiments, optical multiplexer 412 and optical components 416₁ - 416_{N} can be included inside catheter 102 of FIG. 1. In some embodiments, optical multiplexer 412 can be located inside console 110 of FIG. 1 and coupled to optical components 416₁ - 416_{N} in catheter 102 via optical connections 112. In some embodiments, the first optical coupler 404, the second optical coupler 414, and the reference arm 408 can be included in console 110.

Interferometer 410 provides a plurality of optical output signals by second optical coupler 414, each corresponding to one of optical components 416₁ - 416_{N}. Each of the optical output signals includes information about the interference between the reference arm and the sample arm coupled to one of optical components 416₁ - 416_{N}. The optical output signals are detected by an optical detector 422. Optical detector 422 can convert the optical output signals into electrical signals containing information about the interference between the reference arm and the sample arm coupled to one of optical component 416₁ - 416_{N}, as functions of time.

In some embodiments, interferometer 410 can include optical fibers connecting optical coupler 404, optical coupler 414, optical multiplexer 412, and optical components 416₁ - 416_{N}. In some embodiments, interferometer 410 can be connected to coherent light source 402 and optical detector 422 via optical fibers. In some embodiments, the optical fibers can be single mode fibers, multimode fibers, or a combination thereof. In some embodiments, some or all of the optical fibers can be polarization-maintaining (PM) fibers.

Since the wavelength of the beam of coherent light is being swept within a wavelength range under a sweeping frequency, the wavelength is also a function of time. Therefore, within each cycle of the wavelength sweeping, each of the electrical signals provided by optical detector 422 is also a function of the varying wavelength. A Fourier transform of the electrical signal from a wavelength domain (or equivalently, a wavenumber domain) into a real space domain (or equivalently, a depth domain) can provide signals related to optical properties of a location of sample 418 probed by an optical component, as functions of scan depth.

In some embodiments, optical detector 422 can process the optical output signals provided by interferometer 410 for effective measurement. For example, optical detector 422 can amplify the optical output signals, or filter the optical output signals to remove noise. In order to avoid signal distortion and provide high quality electrical signals containing information about sample 418, optical detector 422 should have a sufficient bandwidth to process and detect the optical output signals. In some embodiments, optical detector 422 can have a bandwidth of about 100 MHz.

In some embodiments, optical detector 422 can be located inside console 110 in FIG. 1, and the optical output signals can be transmitted to optical detector 422 via optical connections 112 and interface 116. In some embodiments, optical detector 422 can be located inside catheter 102 in FIG. 1, and the electrical signals provided by optical detector 422 can be transmitted to console 110 via electrical connections 114 and interface 116.

Electrical signals provided by optical detector 422 can further be sampled into digital data by a data acquisition unit 424, at a data sampling rate. In some embodiments, data acquisition unit 424 can be located inside console 110 in FIG. 1. In order to avoid signal distortion or loss of information in the signal, the data sampling rate of data acquisition unit 424 can be sufficiently higher than the bandwidth of the electrical signals provided by optical detector 422. In some embodiments, the data sampling rate can be greater than the bandwidth of optical detector. For example, the data sampling rate can greater than 100 MHz. In some embodiments, the data sampling rate can be between 0 and 175 MHz. In some embodiments, the data sampling rate can be between 0 and 400 MHz. In some embodiments, the data sampling rate of data acquisition unit 424 can be set by the k-clock of coherent light source 402 (for example, the k-clock of a VCSEL swept laser), such that the overall image range can automatically be ensured, and the sweeping nonlinearity can be compensated.

The data acquired by data acquisition unit 424 is further transmitted to data processing unit 426. In some embodiments, data processing unit 426 can be located in console 110 in FIG. 1. In some embodiments, data processing unit 426 can be a processing unit of computer 428. Data processing unit 426 can process the data and extract useful information from the data that can help assisting the analysis of the condition of sample 418. For example, data processing unit 426 can use fast Fourier transform to convert the data as a function of wavelength into a plurality of signals as functions of scan depth from the surface of sample 418, with each of the plurality of signals corresponding to the condition of a location of sample 418 measured by one of the optical components 416₁ - 416_{N}. In some embodiments, data processing unit 426 can also be connected to coherent light source 402 to acquire the phase of the sweeping wavelength, so that the information of the wavelength is collected by data processing unit 426 for applying fast Fourier transform. In some embodiments, the plurality of signals can include information about refraction, birefringence, polarization, and/or other optical properties of sample 418. The plurality of signals as functions of scan depth can further be transmitted to display on a screen of computer 428.

In some embodiments, data processing unit 426 can also process the plurality of signals to extract information of interest in them. For example, data processing unit 426 can detect one or more reference features in the plurality of signals, and crop regions of interest in the plurality of signals, based on the location of one or more reference features.

In some embodiments, the coherent light delivered to sample 418 via optical components 416₁ - 416_{N} can be linearly polarized. In some embodiments, a polarization of the coherent light delivered to sample 418 can be known by either controlling the polarization of the coherent light or sampling the coherent light and measuring polarization. In some embodiments, information about the polarization of the coherent light can be known. In some embodiments, using PM fibers in system 400, the polarization of the coherent light sent to sample 418 can be fixed and is therefore known. In some embodiments, using single mode (SM) fibers in system 400, the polarization of the coherent light sent to sample 418 is not fixed, but can be computed by normalizing it using a known polarization state measured in system 400. In some embodiments, information about a relative polarization of the coherent light can be known. In some embodiments, information about the polarization or the relative polarization of the coherent light is helpful for extracting the information of a fiber alignment of sample 418 by analyzing the output signals of optical detector 422 using data processing unit 426.

FIG. 4B illustrates an example block diagram showing a full chain of integration of data acquisition of the optical signals, according to aspects of the present disclosure. The data acquisition and processing of the optical signals may be implemented by the console 110. In some aspects, a plurality of single-ended signals together with a reference interferometer for signal processing purposes may be digitalized and read by console 110 in a parallel or a single processing stage. In some aspects, the processing may be implemented at or near real-time (e.g., about 50 ms or similar values) In some aspects, any number of signals may be digitalized. These signals may be combined and processed to measure optical properties of tissue, such as birefringence, tissue stability, dragging speed, and the like. The processed signals may be shown on a graphical user interface (GUI) presented on a display (e.g., display 125). In some aspects, the GUI may be refreshed such that multiple channels of the detector (e.g., 15 channels) may be represented at once. In some aspects, the console 110 may buffer optical data from all or multiple channels, and the data may be refreshed and presented in the GUI once the data has been processed.

In some aspects, data transfer and data processing may be optimized in different software abstraction layers, such that the data integrity is maintained while improving the refresh time in the GUI. Once optical data (e.g., OCT structural/polarization data) has been obtained, tissue detection algorithms using cluster methods or other methods may be implemented to separate tissue from other artifacts.

Furthermore, optimization algorithms may be applied to compensate for non-linearities and phase noise of the optical source while switching using an external reference interferometer at different optical path delays. In some aspects, additional auto-calibration methods may be implemented to optimize polarization states without manual interaction and auto-adjust optical fiber lengths using motors and retroreflectors, such that the coherence range is optimized for each fiber.

### Example Methods for Mapping Optical Measurements

The catheters, consoles, and systems described herein may be used to perform optical analysis and display results of the optical analysis of tissue. By utilizing the optical analysis described herein, the catheter and optical systems disclosed herein (e.g., catheter system 100 in FIG. 1 and optical system 400 in FIG. 4A) may allow evaluation of a lesion formation in tissue in or near real-time using a three-dimensional model of the tissue.

Various methods and other aspects of catheters and systems described thus far can be implemented, for example, using catheter 200 shown in FIG. 2, system 100 shown in FIG. 1 (including catheter 102 and console 110), and/or optical system 400 shown in FIG. 4A. The methods described herein may be performed before, during, or after an ablation is performed on the tissue.

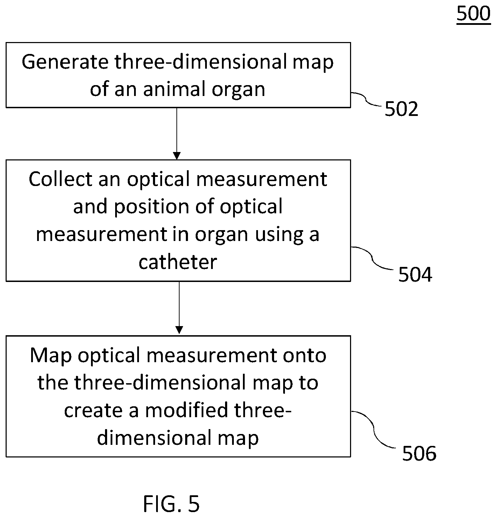

FIG. 5 illustrates an example method 500 for mapping optical measurements on a three-dimensional map, according to aspects of the present disclosure.

At block 502, a three-dimensional map of an animal organ (e.g., a human organ) is generated. In some aspects, the organ may be a heart. It is to be appreciated that the methods described herein may be performed on any tissue that has properties of birefringence. The three-dimensional map may be generated by sweeping the tip of a catheter (e.g., catheter 100) on all sides of the organ cavity to retrieve coordinates of the organ. The collected data of the organ is then transmitted to a system that includes hardware (e.g., circuits), firmware, software, or any combination thereof (e.g., computer system 800) that may generate navigable three-dimensional maps.

In some aspects, the three-dimensional map may be generated by system 100. In some aspects, the three-dimensional map may be generated by a separate, or second, system that generates three-dimensional maps of organs. The three-dimensional map is generated such that a user may navigate a catheter through the organ using the three-dimensional map as a reference. For example, the three-dimensional map may be navigable by a catheter by including anatomical plane coordinates, landmark-based coordinates, or any other nonlinear approaches of representing the organ by coordinates. Example methods of generating an electrophysiological map may be found in U.S. Patent Nos. 6,226,542 and 6,892,091, the disclosures of which are incorporated by reference herein.

At block 504, an optical measurement and a position of the optical measurement are collected by a catheter (e.g., catheter 102). The optical measurement may be acquired from the portion of tissue using at least one optical port in the catheter. In some aspects, the optical measurement may include one or more optical coherence tomography (OCT) signals and/or optical coherence reflectometry (OCR) signals acquired from the portion of tissue. In some aspects, the optical measurements may be collected before ablation to allow electrophysiologists to determine where and how the ablation should be performed. In some aspects, the optical measurements may be collected during or after an ablation. During an ablation, the optical measurements may be collected and processed to guide an electrophysiologist and/or obtain real-time or near real-time information regarding the progress of the ablation. After an ablation, the optical measurements may be collected to allow an electrophysiologist to determine the success of the ablation.

The position and/or orientation of the catheter tip may be determined by known methods, such as MRI or based on impedance or use of electromagnetic position sensors in the tip of the catheter that generate an electrical signal when the optical measurement is taken. The optical measurement and position may be collected simultaneously by the catheter. In some aspects, the optical measurement and position may be acquired by the components shown in the block diagram of FIG. 4A, by the optical system 400, and/or by the console 110 in FIG. 1.

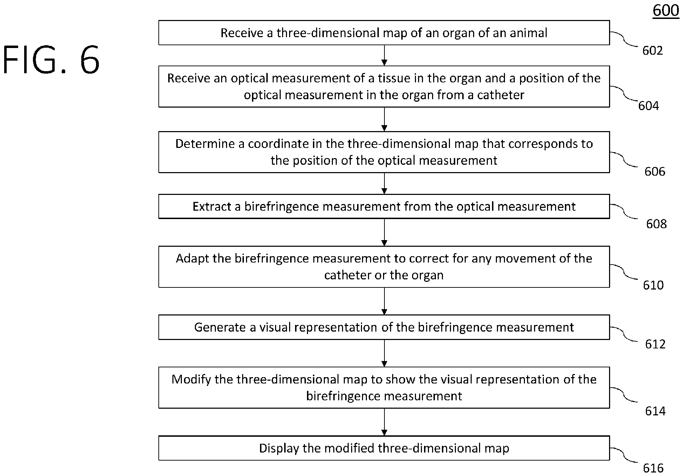

At block 506, the optical measurement is mapped onto the three-dimensional map to create a modified three-dimensional map. Block 506 may be performed by console 110 and display 125 of system 100. Block 506 is further described herein by FIG. 6.

FIG. 6 illustrates an example method 600 for mapping optical measurements, such as birefringence measurements, on a three-dimensional map, according to aspects of the present disclosure. It is to be appreciated that the methods described herein are described in reference to system 100, but any system that includes a processing unit that processes optical and electrical signals may be used to perform method 600. Method 600 may perform in real-time or near real-time such that an electrophysiologist or other user may view the birefringence measurements while the procedure is in progress.

At block 602, a three-dimensional map of an organ of an animal is received. The three-dimensional map may be the three-dimensional map generated in block 502. The three-dimensional map may be received by a computer system (e.g., computer system 800) that is coupled to system 100. The three-dimensional map may be stored in a local memory and/or a patient database. The three-dimensional map may be displayed on display 125 for a user (e.g., electrophysiologist) to view during a procedure.

At block 604, a processing unit may receive an optical measurement of a tissue in the organ and a position of the optical measurement in the organ from catheter 102. In some aspects, the processing unit may be console 110. It is to be appreciated that a plurality of optical measurements and their respective positions may be received. Therefore, block 604 and the following blocks of method 600 may be repetitively performed on a plurality of optical measurements. In some aspects, optical measurements may be received for each position in the organ.

At block 606, the processing unit may determine a coordinate in the three-dimensional map that corresponds to the position of the optical measurement.

At block 608, a birefringence measurement is extracted from the optical measurement. The birefringence measurement of the portion of tissue may be extracted by analyzing the optical measurement using a processing device coupled to the catheter. In some aspects, the one or more optical properties include at least one of polarization or spectral information (e.g., spectroscopic information or imaging information from tissue).

At block 610, the processing unit may adapt the birefringence measurement to correct for movement of the catheter or organ while the measurement was taken. In some aspects, the adapting could be correcting for, modifying, cleaning, reducing noise in, or otherwise digitally processing the birefringence measurements taken with movement. For example, the processing unit may detect one or more reference features in the plurality of signals, and crop regions of interest in the plurality of signals, based on the location of one or more reference features.

At block 612, the processing unit may generate a visual representation of the birefringence measurement. In some aspects, the visual representation may represent a plurality of birefringence measurements taken at the coordinates to account for birefringence measurements taken at different depths of the tissue. In some aspects, the visual representation may include a gradient of colors and a gradient of shades that represent the birefringence measurement. For example, purple may signify tissue with high birefringence at the coordinates and red may signify tissue with low birefringence at the coordinates. As birefringence decreases due to ablation, the colors may fade to represent the decreasing birefringence.

At block 614, the processing unit may modify the three-dimensional map at the coordinates to show the visual representation of the birefringence measurement at the coordinates. The modification may include any of inserting, overlaying, or otherwise replacing the position in the three-dimensional map with the visual representation. The modification creates a modified three-dimensional map that includes the structural and optical properties of the organ. The processing unit may modify the entirety of the three-dimensional map at once based on received birefringence measurements at coordinates across the map, the processing unit may modify portions of the three-dimensional map in real-time or near real-time as updated birefringence measurements are received.

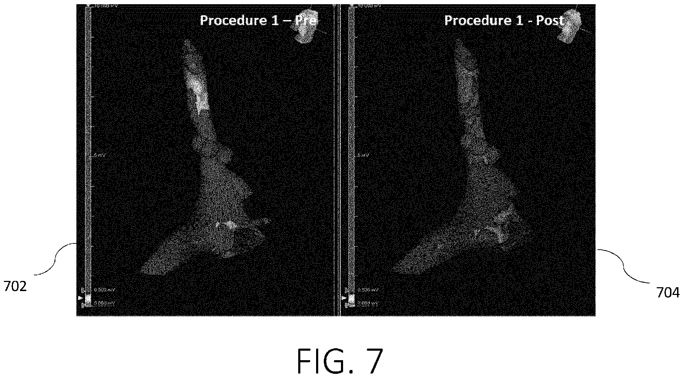

At block 616, the processing unit may transmit the modified three-dimensional map to a computer system and the computer system may display the modified three-dimensional map. The modified three-dimensional map may be used to guide the decisions of location of ablation during an ablation procedure or track the decrease of anisotropy of the tissue induced by an ablation over the whole procedure time. An exemplary modified three-dimensional map is described herein by FIG. 7.

### Example Three-Dimensional Map

FIG. 7 illustrates an example of a modified three-dimensional map that displays birefringence measurements. The example image shows the birefringence measurements of a heart, but it is to be appreciated that the methods described herein may be used on any tissue that has properties of birefringence.

Map 702 displays a modified three-dimensional map of a heart before an ablation procedure. In this example, the purple signifies tissue with high birefringence and red signifies tissue with low birefringence.

Map 704 displays a modified three-dimensional map of the heart after an ablation procedure. As the conduction decreases in the heart at a given position, the color of the heart at that position in the three-dimensional map may fade or change until there is no birefringence detected. In some aspects, optical measurements may be collected at the same position over a specific period of time, which allows a user (e.g., electrophysiologist) to observe the changes in birefringence as the cells die after an ablation. In some aspects, the ablation is successful when the birefringence decreases by a certain amount (e.g., 20%, 30%, or 40%).

### Exemplary Computing Aspects

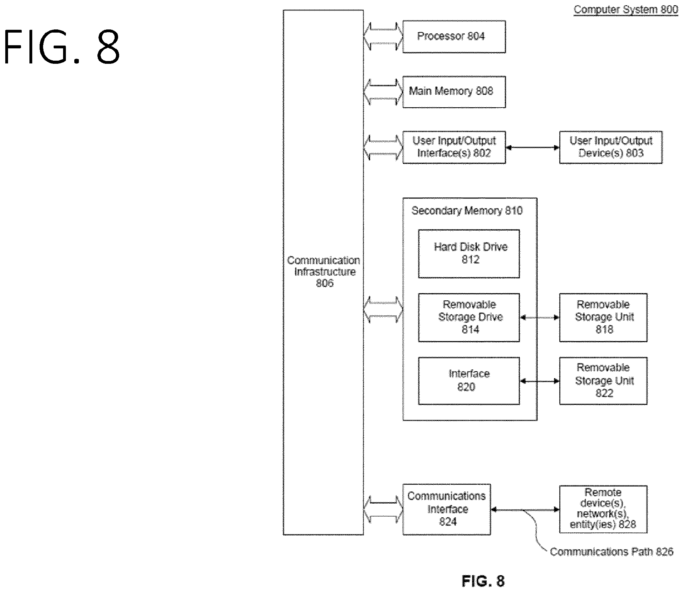

FIG. 8 is a block diagram of example components of computer system 800. One or more computer systems 800 may be used, for example, to implement any of the aspects discussed herein, as well as combinations and sub-combinations thereof. In some aspects, one or more computer systems 800 may be used to implement the method 500 shown in FIG. 5, the method 600 shown in FIG. 6, and/or console 110, signal generator 120, and display 125, as described herein. Computer system 800 may include one or more processors (also called central processing units, or CPUs), such as a processor 804. Processor 804 may be connected to a communication infrastructure or bus 806.

Computer system 800 may also include user input/output interface(s) 802, such as monitors, keyboards, pointing devices, etc., which may communicate with communication infrastructure 806 through user input/output interface(s) 803.

One or more of processors 804 may be a graphics processing unit (GPU). In an aspect, a GPU may be a processor that is a specialized electronic circuit designed to process mathematically intensive applications. The GPU may have a parallel structure that is efficient for parallel processing of large blocks of data, such as mathematically intensive data common to computer graphics applications, images, videos, etc.

Computer system 800 may also include a main or primary memory 808, such as random-access memory (RAM). Main memory 808 may include one or more levels of cache. Main memory 808 may have stored therein control logic (i.e., computer software) and/or data. In some aspects, main memory 808 may include optical logic configured to perform analysis of optical measurements obtained from tissue by a catheter and determine lesion predictions.

Computer system 800 may also include one or more secondary storage devices or memory 810. Secondary memory 810 may include, for example, a hard disk drive 812 and/or a removable storage drive 814.

Removable storage drive 814 may interact with a removable storage unit 818. Removable storage unit 818 may include a computer usable or readable storage device having stored thereon computer software (control logic) and/or data. Removable storage unit 818 may be a program cartridge and cartridge interface (such as that found in video game devices), a removable memory chip (such as an EPROM or PROM) and associated socket, a memory stick and USB port, a memory card and associated memory card slot, and/or any other removable storage unit and associated interface. Removable storage drive 814 may read from and/or write to removable storage unit 818.

Secondary memory 810 may include other means, devices, components, instrumentalities, or other approaches for allowing computer programs and/or other instructions and/or data to be accessed by computer system 800. Such means, devices, components, instrumentalities, or other approaches may include, for example, a removable storage unit 822 and an interface 820. Examples of the removable storage unit 822 and the interface 820 may include a program cartridge and cartridge interface (such as that found in video game devices), a removable memory chip (such as an EPROM or PROM) and associated socket, a memory stick and USB port, a memory card and associated memory card slot, and/or any other removable storage unit and associated interface.

Computer system 800 may further include a communication or network interface 824. Communication interface 824 may enable computer system 800 to communicate and interact with any combination of external devices, external networks, external entities, etc. (individually and collectively referenced by reference number 828). For example, communication interface 824 may allow computer system 800 to communicate with external or remote devices 828 over communications path 826, which may be wired and/or wireless (or a combination thereof), and which may include any combination of LANs, WANs, the Internet, etc. Control logic and/or data may be transmitted to and from computer system 800 via communication path 826. In some aspects, computer system 800 may be coupled to a catheter via a connector and optical and electrical connections at communication interface 824, including optical fibers and electrical wiring, pins, and/or components.

Computer system 800 may also be any of a personal digital assistant (PDA), desktop workstation, laptop or notebook computer, netbook, tablet, smartphone, smartwatch or other wearables, appliance, part of the Internet-of-Things, and/or embedded system, to name a few non-limiting examples, or any combination thereof.

Computer system 800 may be a client or server, accessing or hosting any applications and/or data through any delivery paradigm, including but not limited to remote or distributed cloud computing solutions; local or on-premises software ("on-premise" cloud-based solutions); "as a service" models (e.g., content as a service (CaaS), digital content as a service (DCaaS), software as a service (SaaS), managed software as a service (MSaaS), platform as a service (PaaS), desktop as a service (DaaS), framework as a service (FaaS), backend as a service (BaaS), mobile backend as a service (MBaaS), infrastructure as a service (IaaS), etc.); and/or a hybrid model including any combination of the foregoing examples or other services or delivery paradigms.

Any applicable data structures, file formats, and schemas in computer system 800 may be derived from standards including but not limited to JavaScript Object Notation (JSON), Extensible Markup Language (XML), Yet Another Markup Language (YAML), Extensible Hypertext Markup Language (XHTML), Wireless Markup Language (WML), MessagePack, XML User Interface Language (XUL), or any other functionally similar representations alone or in combination. Alternatively, proprietary data structures, formats or schemas may be used, either exclusively or in combination with known or open standards.

In some aspects, a tangible, non-transitory apparatus or article of manufacture comprising a tangible, non-transitory computer useable or readable medium having control logic (software) stored thereon may also be referred to herein as a computer program product or program storage device. This includes, but is not limited to, computer system 800, main memory 808, secondary memory 810, and removable storage units 818 and 822, as well as tangible articles of manufacture embodying any combination of the foregoing. Such control logic, when executed by one or more data processing devices (such as computer system 800), may cause such data processing devices to operate as described herein.

It is to be appreciated that the Detailed Description section, and not the Summary and Abstract sections, is intended to be used to interpret the claims. The Summary and Abstract sections may set forth one or more but not all exemplary aspects of the present disclosure as contemplated by the inventor(s), and thus, are not intended to limit the present disclosure and the appended claims in any way.

Aspects of the present disclosure have been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific aspects will so fully reveal the general nature of the disclosure that others can, by applying knowledge within the skill of the art, readily modify and/or adapt for various applications such specific aspects, without undue experimentation, without departing from the general concept of the present disclosure. Therefore, such adaptations and modifications are intended to be within the meaning and range of equivalents of the disclosed aspects, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the present disclosure should not be limited by any of the above-described exemplary aspects, but should be defined only in accordance with the following claims and their equivalents.

Furthermore, the following aspects are explicitly disclosed:

Aspect 1: A method, comprising: receiving, by one or more processors, a three-dimensional map of an organ of an animal, wherein the three-dimensional map comprises coordinates; receiving, by the one or more processors, an optical measurement of a tissue in the organ and a position of the optical measurement in the organ from a catheter comprising an optical fiber; determining, by the one or more processors, a coordinate in the three-dimensional map that corresponds to the position of the optical measurement; extracting, by the one or more processors, a birefringence measurement from the optical measurement; generating, by the one or more processors, a visual representation of the birefringence measurement; and modifying, by the one or more processors, the three-dimensional map at the coordinate to show the visual representation of the birefringence measurement at the coordinate, the modifying creating a modified three-dimensional map.

Aspect 2: The method of aspect 1, further comprising: displaying, by the one or more processors, the modified three-dimensional map on a computing device.

Aspect 3: The method of aspect 1, further comprising: adapting, by the one or more processors, the birefringence measurement to correct for any movement of the catheter; and adapting, by the one or more processors, the birefringence measurement to correct for any movement of the organ.

Aspect 4: The method of aspect 1, wherein the visual representation of the birefringence measurement includes a gradient of colors and a gradient of shades applied to the three-dimensional map.

Aspect 5: The method of aspect 1, wherein the optical measurement is a first optical measurement, the method further comprising: receiving, by the one or more processors, a second optical measurement of a tissue in the organ and a second position, the second position being a position of the second optical measurement in the organ from the catheter; determining, by the one or more processors, a second coordinate in the three-dimensional map that corresponds to the second position; extracting, by the one or more processors, a second birefringence measurement from the second optical measurement; generating, by the one or more processors, a second visual representation, the second visual representation being of the second birefringence measurement; and modifying, by the one or more processors, the modified three-dimensional map at the second coordinate to show the second visual representation at the second coordinate.

Aspect 6: The method of aspect 1, wherein the visual representation represents birefringence measurements at a plurality of depths of the tissue at the position.

Aspect 7: A system comprising: a catheter, wherein the catheter collects an optical measurement of a portion of a tissue in an organ of an animal and a position of the optical measurement in the organ; a plurality of optical fibers located within the catheter; and a computing device coupled to the plurality of optical fibers through a connector, the computing device comprising a processor and a memory having instructions stored thereon that, when executed by the processor, cause the processor to: receive, by the processor, a three-dimensional map of the organ, wherein the three-dimensional map comprises coordinates; receive, from the optical fibers, the optical measurement collected from the catheter; receive, from the position sensor, the position of the optical measurement collected from the catheter; determine, by the processor, a coordinate in the three-dimensional map that corresponds to the position of the optical measurement; extract, by the processor, a birefringence measurement from the optical measurement; generate, by the processor, a visual representation of the birefringence measurement; and modify, by the processor, the three-dimensional map at the coordinate to show the visual representation of the birefringence measurement at the coordinate, the modifying creating a modified three-dimensional map.

Aspect 8: The system of aspect 7, the instructions further causing the processor to: display, by the processor, the modified three-dimensional map on the computing device.

Aspect 9: The system of aspect 7, the instructions further causing the processor to: adapt, by the processor, the birefringence measurement to correct for any movement of the catheter; and adapt, by the processor, the birefringence measurement to correct for any movement of the organ.

Aspect 10: The system of aspect 7, wherein the visual representation of the birefringence measurement includes a gradient of colors and a gradient of shades applied to the three-dimensional map.

Aspect 11: The system of aspect 7, wherein the optical measurement is a first optical measurement, the instructions further causing the processor to: receive, by the processor, a second optical measurement of a tissue in the organ and a second position, the second position being a position of the second optical measurement in the organ from the catheter; determine, by the processor, a second coordinate in the three-dimensional map that corresponds to the second position; extract, by the processor, a second birefringence measurement from the second optical measurement; generate, by the processor, a second visual representation, the second visual representation being of the second birefringence measurement; and modify, by the processor, the modified three-dimensional map at the second coordinate to show the second visual representation at the second coordinate.

Aspect 12: The system of aspect 7, wherein the visual representation represents birefringence measurements at a plurality of depths of the tissue at the position.

Aspect 13: The system of aspect 7, wherein the catheter comprises a distal section, a proximal section, and a sheath coupled between the proximal section and the distal section, wherein the distal section comprises a tip with a plurality of optical ports and a position sensor, wherein the optical ports collect the optical measurement and the position sensor collects the position.

Aspect 14: A non-transitory computer-readable device having instructions stored thereon that, when executed by at least one computing device, cause the at least one computing device to perform operations comprising: receiving a three-dimensional map of an organ of an animal, wherein the three-dimensional map comprises coordinates; receiving an optical measurement of a tissue in the organ and a position of the optical measurement in the organ from a catheter comprising an optical fiber; determining a coordinate in the three-dimensional map that corresponds to the position of the optical measurement; extracting a birefringence measurement from the optical measurement; generating a visual representation of the birefringence measurement; and modifying the three-dimensional map at the coordinate to show the visual representation of the birefringence measurement at the coordinate, the modifying creating a modified three-dimensional map.

Aspect 15: The non-transitory computer readable device of aspect 14, the operations further comprising: displaying the modified three-dimensional map on a computing device.

Aspect 16: The non-transitory computer-readable device of aspect 14, the operations further comprising: adapting the birefringence measurement to correct for any movement of the catheter; and adapting the birefringence measurement to correct for any movement of the organ.

Aspect 17: The non-transitory computer-readable device of aspect 14, wherein the visual representation of the birefringence measurement includes a gradient of colors and a gradient of shades applied to the three-dimensional map.

Aspect 18: The non-transitory computer-readable device of aspect 14, wherein the optical measurement is a first optical measurement, the operations further comprising: receiving a second optical measurement of a tissue in the organ and a second position, the second position being a position of the second optical measurement in the organ from the catheter; determining a second coordinate in the three-dimensional map that corresponds to the second position; extracting a second birefringence measurement from the second optical measurement; generating a second visual representation, the second visual representation being of the second birefringence measurement; and modifying the modified three-dimensional map at the second coordinate to show the second visual representation at the second coordinate.

Aspect 19: The non-transitory computer-readable device of aspect 14, wherein the visual representation represents birefringence measurements at a plurality of depths of the tissue at the position.

Aspect 20: The non-transitory computer-readable device of aspect 14, wherein the catheter comprises a distal section, a proximal section, and a sheath coupled between the proximal section and the distal section, wherein the distal section comprises a tip with a plurality of optical ports and a position sensor, wherein the optical ports collect the optical measurement and the position sensor collects the position.

## Claims

1. A method, comprising:
receiving, by one or more processors, a three-dimensional map of an organ of an animal, wherein the three-dimensional map comprises coordinates;
receiving, by the one or more processors, an optical measurement of a tissue in the organ and a position of the optical measurement in the organ from a catheter comprising an optical fiber;
determining, by the one or more processors, a coordinate in the three-dimensional map that corresponds to the position of the optical measurement;
extracting, by the one or more processors, a birefringence measurement from the optical measurement;
generating, by the one or more processors, a visual representation of the birefringence measurement; and
modifying, by the one or more processors, the three-dimensional map at the coordinate to show the visual representation of the birefringence measurement at the coordinate, the modifying creating a modified three-dimensional map.

2. The method of claim 1, further comprising:
displaying, by the one or more processors, the modified three-dimensional map on a computing device.

3. The method of claim 1, further comprising:
adapting, by the one or more processors, the birefringence measurement to correct for any movement of the catheter; and
adapting, by the one or more processors, the birefringence measurement to correct for any movement of the organ.

4. The method of claim 1, wherein the visual representation of the birefringence measurement includes a gradient of colors and a gradient of shades applied to the three-dimensional map.

5. The method of claim 1, wherein the optical measurement is a first optical measurement, the method further comprising:
receiving, by the one or more processors, a second optical measurement of a tissue in the organ and a second position, the second position being a position of the second optical measurement in the organ from the catheter;
determining, by the one or more processors, a second coordinate in the three-dimensional map that corresponds to the second position;
extracting, by the one or more processors, a second birefringence measurement from the second optical measurement;
generating, by the one or more processors, a second visual representation, the second visual representation being of the second birefringence measurement; and
modifying, by the one or more processors, the modified three-dimensional map at the second coordinate to show the second visual representation at the second coordinate.

6. The method of claim 1, wherein the visual representation represents birefringence measurements at a plurality of depths of the tissue at the position.

7. A system comprising:
a catheter, wherein the catheter collects an optical measurement of a portion of a tissue in an organ of an animal and a position of the optical measurement in the organ;
a plurality of optical fibers located within the catheter;
a position sensor located within the catheter; and
a computing device coupled to the plurality of optical fibers through a connector, the computing device comprising a processor and a memory having instructions stored thereon that, when executed by the processor,
cause the processor to:
receive, by the processor, a three-dimensional map of the organ, wherein the three-dimensional map comprises coordinates;
receive, from the optical fibers, the optical measurement collected from the catheter;
receive, from the position sensor, a position of the optical measurement collected from the catheter;
determine, by the processor, a coordinate in the three-dimensional map that corresponds to the position of the optical measurement;
extract, by the processor, a birefringence measurement from the optical measurement;
generate, by the processor, a visual representation of the birefringence measurement; and
modify, by the processor, the three-dimensional map at the coordinate to show the visual representation of the birefringence measurement at the coordinate, the modifying creating a modified three-dimensional map.

8. The system of claim 7, the instructions further causing the processor to:
display, by the processor, the modified three-dimensional map on the computing device.

9. The system of claim 7, the instructions further causing the processor to:
adapt, by the processor, the birefringence measurement to correct for any movement of the catheter; and
adapt, by the processor, the birefringence measurement to correct for any movement of the organ.

10. The system of claim 7, wherein the visual representation of the birefringence measurement includes a gradient of colors and a gradient of shades applied to the three-dimensional map.

11. The system of claim 7, wherein the optical measurement is a first optical measurement, the instructions further causing the processor to:
receive, by the processor, a second optical measurement of a tissue in the organ and a second position, the second position being a position of the second optical measurement in the organ from the catheter;
determine, by the processor, a second coordinate in the three-dimensional map that corresponds to the second position;
extract, by the processor, a second birefringence measurement from the second optical measurement;
generate, by the processor, a second visual representation, the second visual representation being of the second birefringence measurement; and modify, by the processor, the modified three-dimensional map at the second coordinate to show the second visual representation at the second coordinate.

12. The system of claim 7, wherein the visual representation represents birefringence measurements at a plurality of depths of the tissue at the position.

13. The system of claim 7, wherein the catheter comprises a distal section, a proximal section, and a sheath coupled between the proximal section and the distal section, wherein the distal section comprises a tip with a plurality of optical ports and a position sensor, wherein the optical ports collect the optical measurement and the position sensor collects the position.

14. A non-transitory computer-readable device having instructions stored thereon that, when executed by at least one computing device, cause the at least one computing device to perform operations comprising:
receiving a three-dimensional map of an organ of an animal, wherein the three-dimensional map comprises coordinates;
receiving an optical measurement of a tissue in the organ and a position of the optical measurement in the organ from a catheter comprising an optical fiber;
determining a coordinate in the three-dimensional map that corresponds to the position of the optical measurement;
extracting a birefringence measurement from the optical measurement;
generating a visual representation of the birefringence measurement; and
modifying the three-dimensional map at the coordinate to show the visual representation of the birefringence measurement at the coordinate, the modifying creating a modified three-dimensional map.

15. The non-transitory computer readable device of claim 14, the operations further comprising:
displaying the modified three-dimensional map on a computing device.

16. The non-transitory computer-readable device of claim 14, the operations further comprising:
adapting the birefringence measurement to correct for any movement of the catheter; and
adapting the birefringence measurement to correct for any movement of the organ.

17. The non-transitory computer-readable device of claim 14, wherein the visual representation of the birefringence measurement includes a gradient of colors and a gradient of shades applied to the three-dimensional map.

18. The non-transitory computer-readable device of claim 14, wherein the optical measurement is a first optical measurement, the operations further comprising:
receiving a second optical measurement of a tissue in the organ and a second position, the second position being a position of the second optical measurement in the organ from the catheter;
determining a second coordinate in the three-dimensional map that corresponds to the second position;
extracting a second birefringence measurement from the second optical measurement;
generating a second visual representation, the second visual representation being of the second birefringence measurement; and
modifying the modified three-dimensional map at the second coordinate to show the second visual representation at the second coordinate.

19. The non-transitory computer-readable device of claim 14, wherein the visual representation represents birefringence measurements at a plurality of depths of the tissue at the position.

20. The non-transitory computer-readable device of claim 14, wherein the catheter comprises a distal section, a proximal section, and a sheath coupled between the proximal section and the distal section, wherein the distal section comprises a tip with a plurality of optical ports and a position sensor, wherein the optical ports collect the optical measurement and the position sensor collects the position.
